# EUROPEAN PATENT SPECIFICATION

(11) **EP 2 529 741 B1**
(45) Date of publication and mention of the grant of the patent: **12.02.2014**
(21) Application number: 11168543.4
(22) Date of filing: 01.06.2011
(51) Int. Cl.: A61K 31/513, A61K 9/20, A61K 47/26

(54) **Composition and Tablet Comprising Raltegravir**
Zusammensetzung und Tablette beinhaltend Raltegravir
Composition et comprimé comprenant raltégravir

(43) Date of publication of application: 05.12.2012
(73) Proprietor: Ratiopharm GmbH, 89079 Ulm (DE)
(72) Inventor: Stumm, Daniela, 83730 Fischbachau (DE); Meergans, Dominique, 81477 München (DE)
(74) Representative: Kalhammer, Georg

(56) References cited:
- WO-A1-2011/123754
- WO-A1-2012/137142
- WO-A2-2006/060712
- WO-A2-2007/087188
- MERCK FROSST CANADA: "SUMMARY BASIS OF DECISION (SBD) PrISENTRESS Raltegravir, 400 mg, tablet", 20080807, 7 August 2008 (2008-08-07), pages 1-26, XP007918992,
- GUPTA P ET AL: "Ternary amorphous composites of celecoxib, poly(vinyl pyrrolidone) and meglumine with enhanced solubility", DIE PHARMAZIE, GOVI VERLAG PHARMAZEUTISCHER VERLAG GMBH, ESCHBORN, DE, vol. 60, no. 11, 1 November 2005 (2005-11-01), pages 830-836, XP008098535, ISSN: 0031-7144
- DATABASE BIOSIS [Online] BIOSCIENCES INFORMATION SERVICE, PHILADELPHIA, PA, US; July 2000 (2000-07), DE VILLIERS M M ET AL: "Solubilisation of poorly water soluble non-steroidal anti-inflammatory drugs at low pH with N-methylglucamine", XP002647197, Database accession no. PREV200000414082 & PHARMAZIE, vol. 55, no. 7, July 2000 (2000-07), pages 544-546, ISSN: 0031-7144
- DE VILLIERS M M ET AL: "Solubilisation of poorly water soluble non-steroidal anti-inflammatory drugs at low pH with N-methylglucamine", DIE PHARMAZIE, GOVI VERLAG PHARMAZEUTISCHER VERLAG GMBH, ESCHBORN, DE, vol. 55, no. 7, 1 July 2000 (2000-07-01), pages 544-546, XP008139044, ISSN: 0031-7144

## Description

The present invention relates to a composition and tablet comprising raltegravir and to a process for the preparation of such tablet.

Raltegravir has the chemical name N-(2-(4-(4-fluorobenzylcarbamoyl)-5-hydroxy-1-methyl-6-oxo-1,6-dihydropyrimidine-2-yl)propane-2-yl)-5-methyl-1,3,4-oxydiazol-2-carboxamide and the following chemical structure:

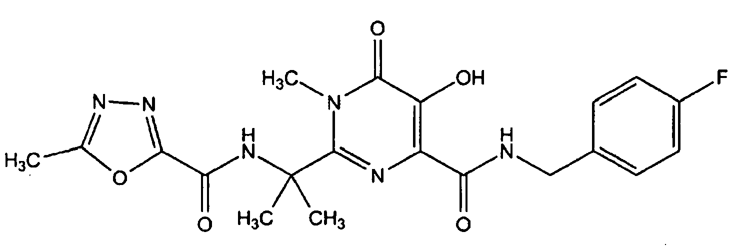

Raltegravir is reported to be useful for inhibiting HIV integrase, prophylaxis of infection by HIV, treating infection by HIV, delaying the onset of AIDS, prophylaxis of AIDS and treating AIDS, in adults, children or infants. Commercially a pharmaceutical composition containing raltegravir is offered by Merck Sharp & Dohme under the trade name Isentress^{®}. In this composition the active ingredient raltegravir is understood to be present as potassium salt.

*N*-Substituted 5-hydroxy-6-oxo-1,6-dihydropyrimidine-4-carboxamides and in particular raltegravir are claimed to be disclosed in EP 1 441 735. The preparation of raltegravir is supposed to be apparently described in examples 18 and 19.

WO 2006/060712 and WO 2006/060730 claim to disclose crystalline raltegravir potassium salt as well as an alternative route of synthesis for the preparation of the free acid of raltegravir and its conversion into the crystalline potassium salt.

The crystalline potassium salt of raltegravir can apparently be prepared by reacting raltegravir in the form of a free acid with a base selected from the group consisting of potassium hydroxide, potassium carbonate, potassium bicarbonate, or potassium alkoxide as described in WO 2006/060730. However, due to limited chemical stability of raltegravir under basic conditions, this preparation process suffers from an undesired hydrolysis of raltegravir. The use of excess amounts of base can lead to the formation of hydrolysis products, whereas the use of sub-equivalent amounts of base can unduly restrict the level of conversion resulting in excessive amounts of unreacted raltegravir. In addition, the formation of hydrolysis products can already take place, if locally high concentrations of base with respect to raltegravir are present during the addition of the base.

The presence of these hydrolysis products in the potassium salt of raltegravir is highly undesirable and therefore an additional purification of the obtained potassium salt of raltegravir may be required. This additional purification leads to additional manufacturing costs and, importantly, to a partial loss of valuable raltegravir.

On the other hand, raltegravir in the form of a free acid shows only limited solubility and bioavailability. Therefore, the use of raltegravir as a free acid in pharmaceutical compositions has been limited until now.

Thus, there is a need for pharmaceutical compositions comprising raltegravir as a free acid and, nevertheless, exhibiting good release properties of raltegravir, both, *in vitro* and *in* vivo, whereby improved *in vitro* release properties indicate also improved *in vivo* release properties.

A general approach for increasing the release properties of a pharmaceutical composition is the use of a solubility enhancer, such as Span (sorbitan monooleate), Tween (polysorbate) or collidon (polyvinylpyrrolidone). Alternatively, the size of the active ingredient particles is reduced in order to provide particles having a higher surface area thereby increasing their dissolution rate. However, for raltegravir it turned out that both options did not result in a sufficient improvement of the dissolution rate of such compositions.

Furthermore, dissolution enhancers are generally dissolved and mixed with the active pharmaceutical ingredient by granulation, spray drying or extrusion. These processes, however, have the disadvantage of requiring a drying step which is costly and results in a thermal stress to the active ingredient. Therefore, it is also desirable to provide a composition and a process for the preparation of raltegravir-containing pharmaceutical compositions, which do not require dissolution of any of the ingredients.

It has surprisingly been found that a pharmaceutical composition in the form of an oral pharmaceutical dosage form comprising raltegravir exhibits superior dissolution rate properties of raltegravir if this pharmaceutical composition further contains N-methylglucamine.

Thus, the present invention relates to a composition comprising raltegravir in amorphous or crystalline form and N-methylglucamine, wherein the composition is in the form of an oral pharmaceutical dosage form.

The present invention is based on the finding that the dissolution profile of a pharmaceutical composition comprising raltegravir can be significantly improved upon usage of raltegravir in combination with N-methylglucamine. Further, it has been found that such composition in the form of a tablet can be prepared by direct compression of a mixture of the ingredients or by a method comprising dry granulation processing, although this embodiment is not limited by the method of manufacture.

In the composition of the present invention raltegravir is present in amorphous form or any crystalline form, including solvates and hydrates. Moreover, raltegravir can be present in any salt form, such as raltegravir potassium salt, or in its salt free form as free acid. Preferably, raltegravir is present in its free acid form.

*N*-Methylglucamine (also known as meglumine) has the chemical name (2*R*,3*R*,4*R*,5*S*)-6-methylaminohexane-1,2,3,4,5-pentol and the following chemical structure:

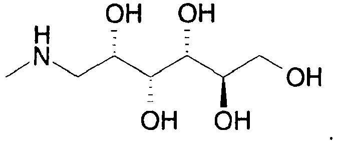

The amount of *N*-methylglucamine in the composition of the present invention is not particularly relevant and can be readily chosen by a person skilled in the art such that it increases the release of the active ingredient from the composition. For example, the weight ratio of raltegravir to N-methylglucamine in the composition of the present invention can be in the range of 5:1 to 1:5, such as in the range of 2:1 to 1:2 and preferably in the range of 1.5:1 to 1:1. The weight ratio can be selected such that from a pharmaceutical composition or dosage form containing raltegravir in free acid form at least 50%, preferably at least 65%, more preferably at least 70% and most preferably at least 80% by weight of the total amount of raltegravir present in the composition or dosage form is dissolved within 1 hour after starting dissolution of the composition or dosage form when tested using 900 ml of water at pH >5, in particular 7.6, in USP apparatus type II (paddle) at 100 rpm and 37°C.

The composition of the present invention may additionally comprise at least one further pharmaceutically acceptable excipient, such as fillers, binding agents, lubricants, flow enhancers, antisticking agents, disintegrating agents, viscosity enhancing agents and wetting agents. As pharmaceutically acceptable excipients, any conventional excipients known to the person skilled in the art may be used.

Preferred examples of the fillers are lactose, mannitol, sorbitol, microcrystalline cellulose or polyoxypropylene polyoxyethylene copolymers (poloxamer 407; e.g. Pluronic^{®}). The filler is suitably present in an amount of 0 to 80 wt.-%, preferably of 0 to 25 wt.-%, more preferably 7 to 21 wt.-% of the total weight of the composition.

The binding agent can for example be microcrystalline cellulose (MCC) or hydroxypropylmethyl cellulose (HPMC). Preferably the binding agent is present in an amount of 1 to 25 wt.-%, more preferably of 3 to 10 wt.-% of the total weight of the composition.

The lubricant and/or antisticking agent may for example be a stearate (e.g. zinc stearate, magnesium stearate) or sodium stearyl fumarate, talc, polytetrafluoroethylene, sodium benzoate, polyethylene glycol 8000, sodium oleate, succinic acid, adipic acid or fumaric acid. The lubricant is suitably present in an amount of 0.1 to 5 wt.-%, preferably of about 1 to 5 wt.-% of the total weight of the composition.

The flow enhancer can for example be colloidal silicon dioxide or talcum. Preferably, the flow enhancer is present in an amount of 0.2 to 8 wt.-%, more preferably of 0.3 to 1 wt.-% of the total weight of the composition.

Preferred disintegrating agents are croscarmellose sodium, sodium carboxymethyl starch, or cross-linked polyvinylpyrrolidone (crospovidone). The disintegrating agent is suitably present in an amount of 0.1 to 20 wt.-%, more preferably at about 0.5 to 7 wt.-% of the total weight of the composition.

If a certain dissolution profile of the pharmaceutical composition is desired it can be advantageous adding a viscosity enhancing agent for delaying the dissolution rate of the composition. Suitable viscosity enhancing agents are for example polysaccharides (e.g. methyl cellulose, carboxymethyl cellulose, hydroxyethyl cellulose, hydroxypropylmethyl cellulose, agar agar, guar gum, tragacanth, alginates, carragenan), proteins (e.g. gelatine), synthetic polymers such as polyvinyl derivatives, polyacrylic acid polymers (e.g. Carbopol 971P, Carbopol 71G or Carbopol 974P), non polymeric gel forming substances (e.g. silicon dioxide) or glycerine. The viscosity enhancing agent can be present in an amount of 0.5 to 10 wt.%, preferably of 1 to 7 wt.-% of the total weight of the composition.

The composition of the present invention can be in the form of the above described powder. Alternatively, the composition can be in the form of a granulate which can, for example, be prepared from the above described powder by usual granulation methods, preferably by compaction, in particular dry granulation.

The pharmaceutical composition of the present invention has a particularly advantageous *in vitro* release profile. The release profile can be determined by using 900 ml of water having a pH >5, in particular 7.6, in the USP apparatus type II with paddle speed 100 rpm at 37°C. Under this condition the pharmaceutical oral dosage form of the present invention allows an amount of at least 50 wt.-%, preferably at least 65 wt.-%, particularly preferred at least 70 wt.-% and most preferred at least 80% of the raltegravir present in the pharmaceutical composition to be released within the first 1 hour.

The oral pharmaceutical dosage form of the present invention can for example be in the form of a tablet or a capsule. The capsule can contain the above described granulate alone or together with further pharmaceutically acceptable ingredients in a capsule, such as a soft or hard gelatine capsule. The tablet preferably is obtainable by direct compression or dry granulation.

The oral pharmaceutical dosage form of the present invention may contain 1.0 to 1000 mg of the active ingredient, raltegravir, preferably 300 mg to 500 mg, such as in particular 400 mg. A preferred dosage of raltegravir for adult humans is oral administration in the form of capsules or tablets in an amount of from 100 mg to 600 mg twice per day. The specific dosage level and frequency of dosage for any particular patient can vary and will depend upon a variety of factors including the patient's age, body weight, general health, sex, diet, the mode and time of administration, rate of excretion, drug combination, and the severity of the particular condition.

In a further embodiment the present invention relates to a process for the preparation of the above described tablet. In one embodiment this process comprises the steps of mixing raltegravir, *N*-methylglucamine and optionally one or more pharmaceutically acceptable excipients to obtain a powder and preparing the tablet by direct compression of said powder.

In a different embodiment this process comprises the steps of mixing raltegravir, *N-*methylglucamine and optionally one or more pharmaceutically acceptable excipients to obtain a powder, preparing a granulate by compaction, preferably dry granulation, of said powder, optionally mixing said granulate with one or more further pharmaceutically acceptable excipients and pressing said granulate and optionally one or more further pharmaceutically acceptable excipients into the tablet.

The tablet of the present invention may have one or more coatings.

Finally, the present invention also relates to the use of *N*-methylglucamine for accelerating the release of raltegravir present in its free acid form from a raltegravir-containing oral pharmaceutical dosage form.

### Figures

Figure 1 shows the dissolution profiles of the tablets obtained in Examples 1 and 2.
Figure 2 shows the dissolution profile of the tablets obtained in Example 1 as well as the dissolution profiles of the compositions obtained in Examples 9 and 10 in comparison to the dissolution profile of Isentress^{®} 400 mg tablets.

The invention will now be further illustrated by the following examples which are not intended to be construed as limiting.

In the examples the amounts of raltegravir, *N*-methylglucamine and further excipients given in the tables refer to the contents per single tablet.

The dissolution profiles were measured in 900 ml of water at pH 7.6 and 37°C using a USP paddle apparatus II at 100 rpm.

### Example 1

**Table 1**

| | |
|---|---|
| Raltegravir | 400.00 mg |
| *N*-Methylglucamine | 300.00 mg |
| Carbopol^{®} 971 P | 10.00 mg |
| Poloxamer 407 | 136.30 mg |
| Magnesium stearate | 26.00 mg |
| Magnesium stearate | 15.00 mg |
| Silica dioxide | 2.70 mg |

A mixture of raltegravir free acid, *N*-methylglucamine, Carbopol^{®} 971 P, Poloxamer 407, silica dioxide and magnesium stearate was prepared in a free fall mixer (Turbula TB 10) for 10 min and sieved through a 1000 µm sieve. Subsequently, the mixture was compacted and granulated to the desired particle size upon using a 1 mm rasping screen (Comil U5). The remaining magnesium stearate was added to the obtained granulate and the material was mixed again in the free fall mixer for 3 min. This mixture was compressed to tablets with an eccentric press (Korsch EK0). The hardness of the prepared tablets was 60 - 120 N.

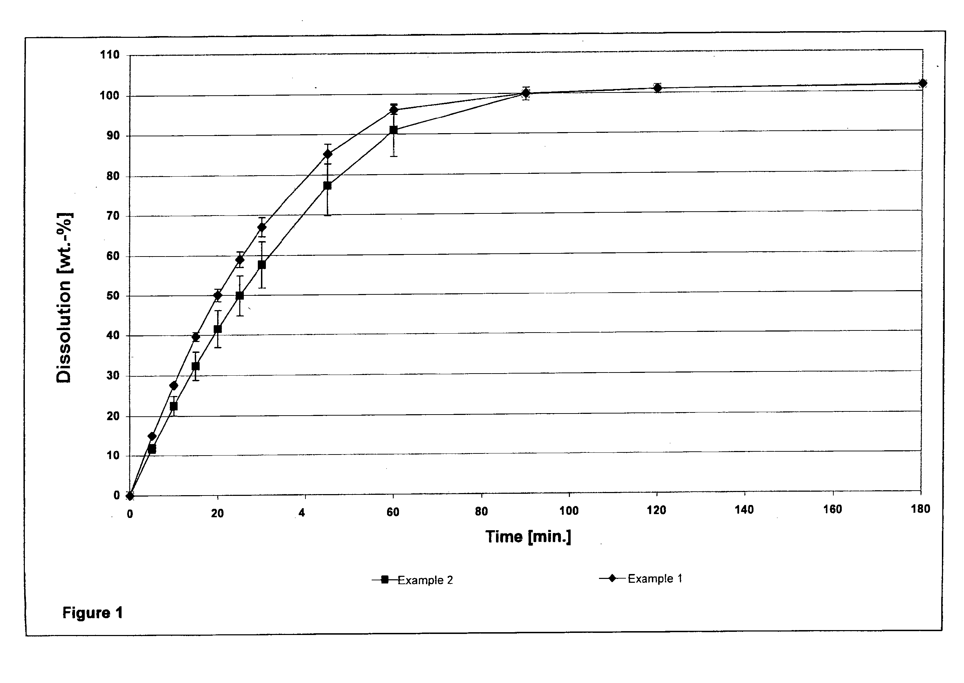

The dissolution of the tablets in water at pH 7.6 is fast and complete. The dissolution profile of the tablets of Example 1 is shown in Figure 1.

### Example 2

**Table 2**

| | |
|---|---|
| Raltegravir | 400.00 mg |
| *N*-Methylglucamine | 300.00 mg |
| Carbopol^{®} 974P | 12.00 mg |
| Poloxamer 407 | 134.30 mg |
| Magnesium stearate | 26.00 mg |
| Magnesium stearate | 15.00 mg |
| Silica dioxide | 2.70 mg |

A mixture of raltegravir free acid, *N*-methylglucamine, Carbopol^{®} 974P, Poloxamer 407, silica dioxide and magnesium stearate was prepared in a free fall mixer (Turbula TB 10) for 10 min and sieved through a 1000 µm sieve. Subsequently, the mixture was compacted and granulated to the desired particle size upon using a 1 mm rasping screen (Comil U5). The remaining magnesium stearate was added to the obtained granulate and the material was mixed again in the free fall mixer for 3 min. This mixture was compressed to tablets with an eccentric press (Korsch EK0). The hardness of the prepared tablets was 60 - 120 N.

The dissolution of the tablets in water at pH 7.6 is fast and complete. The dissolution profile of the tablets of Example 2 is shown in Figure 1.

### Example 3

**Table 3**

| | |
|---|---|
| Raltegravir | 400.00 mg |
| *N*-Methylglucamine | 385.00 mg |
| Poloxamer 407 | 64.00 mg |
| Magnesium stearate | 26.00 mg |
| Magnesium stearate | 15.00 mg |

A mixture of raltegravir free acid, *N*-methylglucamine, Poloxamer 407 and magnesium stearate was prepared in a free fall mixer (Turbula TB 10B) for 10 min and sieved through a 1200 µm sieve. Subsequently, the mixture was compacted and granulated to the desired particle size upon using a 1 mm rasping screen (Comil U5). The remaining magnesium stearate was added to the obtained granulate and the material was mixed again in the free fall mixer for 3 min. This mixture was compressed to tablets with an eccentric press (Korsch EK0).

The dissolution of the prepared tablets in water at pH 7.6 is fast, complete and superior in comparison to the reference (Isentress^{®} 400 mg tablets).

### Example 4

**Table 4**

| | |
|---|---|
| Raltegravir | 400.00 mg |
| *N*-Methylglucamine | 300.00 mg |
| HPMC | 35.00 mg |
| Poloxamer 407 | 114.00 mg |
| Magnesium stearate | 26.00 mg |
| Magnesium stearate | 15.00 mg |

A mixture of raltegravir free acid, *N*-methylglucamine, HPMC, Poloxamer 407 and magnesium stearate was prepared in a free fall mixer (Turbula TB 10B) for 10 min and sieved through a 1200 µm sieve. Subsequently, the mixture was compacted and granulated to the desired particle size upon using a 1 mm rasping screen (Comil U5). The remaining magnesium stearate was added to the obtained granulate and the material was mixed again in the free fall mixer for 3 min. This mixture was compressed to tablets with an eccentric press (Korsch EK0).

The dissolution of the prepared tablets in water at pH 7.6 is fast, complete and superior in comparison to the reference (Isentress^{®} 400 mg tablets).

### Example 5

**Table 5**

| | |
|---|---|
| Raltegravir | 400.00 mg |
| *N*-Methylglucamine | 385.00 mg |
| Poloxamer 407 | 64.00 mg |
| Magnesium stearate | 26.00 mg |

A mixture of raltegravir free acid, *N*-methylglucamine, Poloxamer 407 and magnesium stearate was prepared in a free fall mixer (Turbula TB 10B) for 10 min and sieved through a 1200 µm sieve. This mixture was directly compressed to tablets with an eccentric press (Korsch EK0).

The dissolution of the prepared tablets in water at pH 7.6 is fast, complete and superior in comparison to the reference (Isentress^{®} 400 mg tablets).

### Example 6

**Table 6**

| | |
|---|---|
| Raltegravir | 400.00 mg |
| *N*-Methylglucamine | 300.00 mg |
| Poloxamer 407 | 181.00 mg |
| Magnesium stearate | 9.00 mg |
| Magnesium stearate | 20.00 mg |

A mixture of raltegravir free acid, *N*-methylglucamine, poloxamer 407 and magnesium stearate was prepared, the additional amount of magnesium stearate was added and the resulting mixture was compressed into tablets.

### Example 7

| | |
|---|---|
| Raltegravir | 400.00 mg |
| *N-*Methylglucamine | 300.00 mg |
| Carbopol^{®} 71 G | 20.00 mg |
| Poloxamer 407 | 181.00 mg |
| Magnesium stearate | 9.00 mg |

### Example 7A:

The above ingredients were mixed and the resulting mixture was directly compressed into tablets.

### Example 7B:

The above ingredients were mixed and the resulting mixture was dry granulated. The obtained granulate was then pressed into tablets.

### Example 8

Tablets were prepared using dry granulation of the following ingredients:

| | |
|---|---|
| Raltegravir | 400.00 mg |
| *N*-Methylglucamine | 300.00 mg |
| Carbopol^{®} 71 G | 20.00 mg |
| Poloxamer 407 | 126.30 mg |
| Magnesium stearate | 26.00 mg |
| Magnesium stearate | 15.00 mg |
| Silica dioxide | 2.70 mg |

| | |
|---|---|
| Raltegravir | 400.00 mg |
| *N*-Methylglucamine | 300.00 mg |
| Carbopol^{®} 974P | 12.00 mg |
| Poloxamer 407 | 134.30 mg |
| Magnesium stearate | 26.00 mg |
| Magnesium stearate | 15.00 mg |
| Silica dioxide | 2.70 mg |

| | |
|---|---|
| Raltegravir | 400.00 mg |
| *N*-Methylglucamine | 300.00 mg |
| HPMC | 70.00 mg |
| Poloxamer 407 | 79.00 mg |
| Magnesium stearate | 26.00 mg |
| Magnesium stearate | 15.00 mg |
| Silica dioxide | 3.00 mg |

Tablets were prepared by direct compression of a powder mixture containing the following ingredients:

| | |
|---|---|
| Raltegravir | 400.00 mg |
| *N*-Methylglucamine | 300.00 mg |
| HPMC | 70.00 mg |
| Poloxamer 407 | 91.00 mg |
| Magnesium stearate | 26.00 mg |
| Silica dioxide | 3.00 mg |

### Example 9

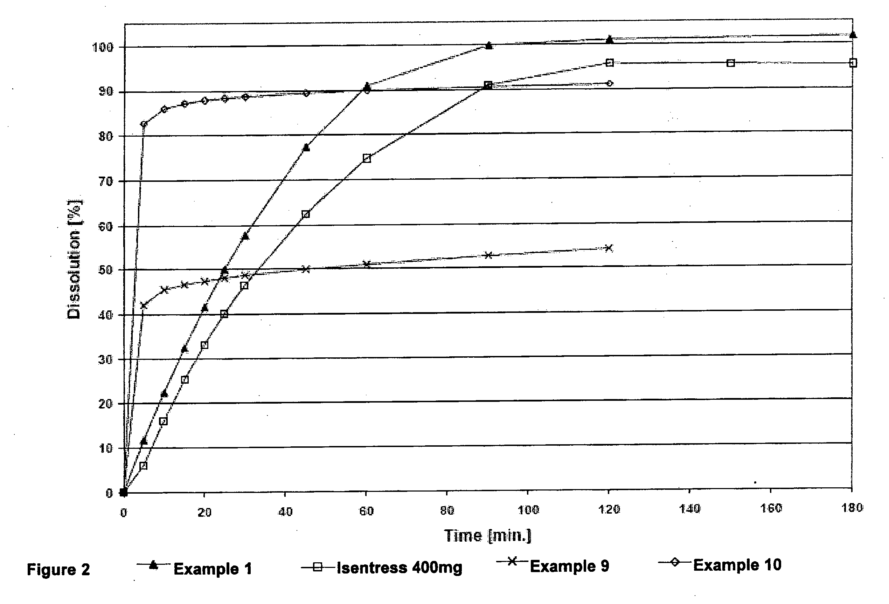

Raltegravir free acid was mixed with N-methylglucamine in the weight ratio of 2:1. The dissolution profile of the obtained composition is shown in Figure 2.

### Example 10

Raltegravir free acid was mixed with N-methylglucamine in the weight ratio of 1:1. The dissolution profile of the obtained composition is shown in Figure 2.

The dissolution profiles of the compositions obtained in examples 9 and 10 demonstrate the advantageous effect of N-methylglucamine on the dissolution rate of raltegravir.

## Claims

1. Composition comprising raltegravir in amorphous or crystalline form and *N-*methylglucamine, wherein the composition is in the form of an oral pharmaceutical dosage form.

2. Composition according to claim 1, wherein the raltegravir is present in its free acid form.

3. Composition according to claim 1 or 2, wherein the weight ratio of raltegravir to N-methylglucamine is in the range of 5:1 to 1:5, preferably in the range of 1.5:1 to 1:1.

4. Composition according to any of claims 1-3, further comprising a viscosity enhancing agent.

5. Composition according to claim 4, where in the viscosity enhancing agent is a polyacrylic acid polymer.

6. Composition according to any of claims 1-5, further comprising one or more pharmaceutically acceptable excipients.

7. Composition according to any of claims 1-6 in the form of a granulate.

8. Composition according to any of claims 1-7, which is a tablet or a capsule.

9. Tablet according to claim 8, which is obtainable by direct compression or dry granulation.

10. Process for the preparation of the tablet according to claim 9, comprising the steps of mixing raltegravir, *N*-methylglucamine and optionally one or more pharmaceutically acceptable excipients to obtain a powder and preparing the tablet by direct compression of said powder.

11. Process for the preparation of the tablet according to claim 9, comprising the steps of mixing raltegravir, *N*-methylglucamine and optionally one or more pharmaceutically acceptable excipients to obtain a powder, preparing a granulate by compaction, preferably dry granulation, of said powder, optionally mixing said granulate with one or more further pharmaceutically acceptable excipients and pressing said granulate and optionally one or more further pharmaceutically acceptable excipients into the tablet.

12. *N*-methylglucamine for use in a method for treatment by therapy wherein the use is accelerating the in vivo rate of dissolution of raltegravir present in its free acid form from a raltegravir-containing oral pharmaceutical dosage form into an aqueous medium.

13. Use of *N*-methylglucamine for accelerating the in vitro rate of dissolution of raltegravir present in its free acid form from a raltegravir-containing oral pharmaceutical dosage form into an aqueous medium.

## Patentansprüche

1. Zusammensetzung, umfassend Raltegravir in amorpher oder kristalliner Form und *N*-Methylglucamin, wobei die Zusammensetzung in Form einer oralen pharmazeutischen Dosierform vorliegt.

2. Zusammensetzung nach Anspruch 1, wobei das Raltegravir in seiner freien Säureform vorliegt.

3. Zusammensetzung nach Anspruch 1 oder 2, wobei das Gewichtsverhältnis von Raltegravir zu N-Methylglucamin im Bereich von 5 : 1 bis 1 : 5, vorzugsweise im Bereich von 1,5 : 1 bis 1 : 1 liegt.

4. Zusammensetzung nach einem der Ansprüche 1 - 3, ferner umfassend ein Mittel zur Erhöhung der Viskosität.

5. Zusammensetzung nach Anspruch 4, wobei das Mittel zur Erhöhung der Viskosität ein Polyacrylsäurepolymer ist.

6. Zusammensetzung nach einem der Ansprüche 1 - 5, ferner umfassend einen oder mehrere pharmazeutisch akzeptable Hilfsstoffe.

7. Zusammensetzung nach einem der Ansprüche 1 - 6 in Form eines Granulats.

8. Zusammensetzung nach einem der Ansprüche 1 - 7, welche eine Tablette oder eine Kapsel ist.

9. Tablette nach Anspruch 8, welche durch direkte Kompression oder Trockengranulation erhältlich ist.

10. Verfahren zur Herstellung der Tablette nach Anspruch 9, umfassend die Schritte Mischen von Raltegravir, *N*-Methylglucamin und gegebenenfalls einem oder mehreren pharmazeutisch akzeptablen Hilfsstoffen unter Erhalt eines Pulvers und Herstellen der Tablette durch direkte Kompression des Pulvers.

11. Verfahren zur Herstellung der Tablette nach Anspruch 9, umfassend die Schritte Mischen von Raltegravir, *N*-Methylglucamin und gegebenenfalls einem oder mehreren pharmazeutisch akzeptablen Hilfsstoffen unter Erhalt eines Pulvers, Herstellen eines Granulats durch Verdichten, vorzugsweise durch Trockengranulation, des Pulvers, gegebenenfalls Mischen des Granulats mit einem oder mehreren weiteren pharmazeutisch akzeptablen Hilfsstoffen und Pressen des Granulats und gegebenenfalls eines oder mehrerer weiterer pharmazeutisch akzeptabler Hilfsstoffe zu der Tablette.

12. *N*-Methylglucamin zur Verwendung in einem Verfahren zur therapeutischen Behandlung, wobei die Verwendung die In-vivo-Auflösungsgeschwindigkeit von Raltegravir, das in seiner freien Säureform vorliegt, aus einer Raltegravir-haltigen oralen pharmazeutischen Dosierform in einem wässerigen Medium beschleunigt.

13. Verwendung von *N*-Methylglucamin zur Beschleunigung der In-vivo-Auflösungsgeschwindigkeit von Raltegravir, das in seiner freien Säureform vorliegt, aus einer Raltegravir-haltigen oralen pharmazeutischen Dosierform in einem wässerigen Medium.

## Revendications

1. Composition comprenant du raltégravir sous forme amorphe ou cristalline et de la N-méthylglucamine, dans laquelle la composition est sous une forme de dosage pharmaceutique oral.

2. Composition selon la revendication 1, dans laquelle le raltégravir est présent sous sa forme d'acide libre.

3. Composition selon la revendication 1 ou 2, dans laquelle la proportion pondérale en poids du raltégravir par rapport à la N-méthylglucamine est compris dans la plage allant de 5 :1 à 1 :5, de préférence dans la plage allant de 1,5 :1 à 1 :1.

4. Composition selon d'une quelconque des revendications 1 à 3, comprenant en outre un agent améliorant la viscosité.

5. Composition selon la revendication 4, dans laquelle l'agent améliorant la viscosité est un polymère d'acide polyacrylique.

6. Composition selon l'une quelconque des revendications 1 à 5, comprenant en outre un ou plusieurs excipients pharmaceutiquements acceptables.

7. Composition selon l'une quelconque des revendications 1 à 6, sous la forme d'un granulé.

8. Composition selon l'une quelconque des revendications 1 à 7 laquelle est une tablette ou une capsule.

9. Comprimé selon la revendication 8, laquelle est susceptible d'être obtenu par compression directe ou par granulation à sec.

10. Procédé de préparation d'un comprimé selon la revendication 9, comprenant les étapes de mélange de raltégravir, de N-méthylglucamine et éventuellement un ou plusieurs excipients pharmaceutiquements acceptables pour obtenir une poudre et préparer le comprimé par compression directe de ladite poudre.

11. Procédé de préparation d'un comprimé selon la revendication 9, comprenant les étapes de mélange de raltégravir, de N-méthylglucamine et éventuellement d'un ou plusieurs excipients pharmaceutiquements acceptables pour obtenir une poudre, préparer un granulé par compactage, de préférence par granulation à sec, de ladite poudre, éventuellement de mélange dudit granulé avec un ou plusieurs excipients pharmaceutiquements acceptables additionnels et de compression dudit granulé et éventuellement d'un ou plusieurs excipients pharmaceutiquements acceptables additionnels dans la le comprimé.

12. N-méthylglucamine pour une utilisation dans une méthode de traitement par thérapie dans laquelle l'utilisation accélère le taux de dissolution *in vivo* de raltégravir présent sous sa forme d'acide libre au départ d'une forme de dosage pharmaceutique oral comprenant du raltégravir dans un milieu aqueux.

13. Utilisation de N-méthylglucamine pour accélérer le taux de dissolution in vivo de raltégravir présent sous sa forme d'acide libre au départ d'une forme de dosage pharmaceutique oral comprenant du raltégravir dans un milieu aqueux.
